# EUROPEAN PATENT APPLICATION

(11) **EP 4 813 914 A1**
(43) Date of publication of application: **30.09.2026**
(21) Application number: 24894099.1
(22) Date of filing: 15.11.2024
(51) Int. Cl.: C01B 33/12, A61K 6/833, A61K 6/84

(54) **AMORPHOUS SILICA-BASED COMPOSITE OXIDE PARTICULATES, METHOD FOR PRODUCING SAME, DENTAL COMPOSITION, AND BLANK FOR DENTAL CUTTING**

(30) Priority: 22.11.2023 JP 2023197884
(71) Applicant: Tokuyama Dental Corporation, Tokyo 110-0016 (JP)
(72) Inventor: NISHIKAWA, Akira, Tokyo 110-0016 (JP); OYA, Naoyuki, Tokyo 110-0016 (JP)
(74) Representative: Lorenz & Kollegen
(86) International application number: PCT/JP2024/040680
(87) International publication number: WO 2025/110108

(57) **Abstract**

[Object] To provide an irregularly shaped silica composite oxide particulate that is blended for enhancing strength of a dental curable composition and less likely to impair polishing properties of a cured body even if the blending amount thereof is increased.

[Solving Means] When, after hydrolysis of alkyl silicate, a metal alkoxide to be compounded is mixed therewith to prepare a sol, and water is blended in the obtained sol to obtain a wet gel, which is then dried, pulverized, and fired to obtain an irregularly shaped silica composite oxide particulate having an average particle diameter of 1 to 10 µm, conditions for preparing the sol an gel are controlled such that, in a diffraction pattern obtained by measuring X-ray diffraction, a ratio B/A of diffraction intensity B at a peak top of a peak with the highest diffraction intensity among diffraction peaks derived from a compounded metal oxide to diffraction intensity A at a peak top of a halo peak derived from amorphous silica is 0.5 to 0.9.

## Description

### Technical Field

The present invention relates to an irregularly shaped silica composite oxide particulate, a method of producing the same, a dental composition that includes a composite oxide particle thereof, and a dental blank for cutting that includes a to-be-cut portion at least partially formed of a cured body of the dental composition.

### Background Art

Composite materials (of a resin and an inorganic filler) such as a dental curable composition that includes a polymerizable monomer (monomer), an inorganic filler, and a polymerization initiator as main components, and a hybrid resin formed of a cured body thereof are widely used as materials for dental prosthetic treatment. For example, the dental curable composition in an uncured state is used for composite resin restoration and dental cement, and the cured body such as a hybrid resin is used as the material of a portion to be cut in a dental mill blank used to produce a prosthesis such as an inlay and a crown using a CAD/CAM system.

Regarding the dental curable composition, a filler formed of a spherical inorganic particulate and a filler formed of an irregularly shaped inorganic particulate are often used in combination as the inorganic filler from the viewpoints of the fluidity of the composition and the strength of the cured body or the viewpoints of the strength and surface smoothness of the cured body (see Patent Literatures 1 to 3). Here, the spherical inorganic particulate refers to a particulate including an inorganic particle having a spherical shape or a substantially spherical shape, and the irregularly shaped inorganic particulate refers to a particulate in which the individual constituent inorganic particles have non-spherical and irregular shapes (typically have fracture surfaces resulting from crushing or pulverization, often have edges, and have irregular shapes), such as those obtained through crushing processing or pulverization processing in the production process.

For example, regarding a dental curable composition intended for use as a composite resin, Patent Literature 1 discloses that a cured body having high mechanical strength (bending strength) can be obtained by using an irregularly shaped inorganic particulate that is at least partially amorphous, has an average particle diameter of 1 to 9 µm, and includes particles having a particle diameter of 1 to 10 µm occupying 95 weight% or more, and a spherical inorganic particulate having an average particle diameter of 0.08 to 1 µm in combination. Further, Patent Literature 2 describes that a photocurable dental restoration material in which a filler formed of a mixture of a spherical inorganic particulate having an average particle diameter of 0.1 to 5 µm and a (favorably) spherical inorganic particulate having an average particle diameter of 0.01 to 0.1 µm is blended at a high content is excellent in aesthetics and strength and that using a filler further including an irregularly shaped inorganic particulate having an average particle diameter of 1 to 9 µm in addition to the mixture allows the mechanical strength to be significantly improved while maintaining the relatively good surface smoothness (aesthetics) although the physical properties are slightly reduced, thereby making it suitable as a restoration material such as molars where high occlusal pressure is applied.

Further, regarding the dental curable composition intended for use as a raw material of a dental resin material for cutting and machining, Patent Literature 3 describes that a combination of irregular shapes and spherical shapes is preferable for the inorganic filler from the viewpoint of the strength of the cured body.

Note that as the material of the particle constituting such an inorganic particulate, a silica composite oxide such as silica and zirconia, which has a refractive index that can be easily controlled with the composition is often used, because it is easy to adjust the refractive index difference of an inorganic filler to obtain a cured body with transparency. Then, it is known that a spherical particulate formed of a silica composite oxide (hereinafter, also referred to as a "spherical silica composite oxide particulate") and an irregularly shaped particulate (hereinafter, also referred to as an "irregularly shaped silica composite oxide particulate") are obtained by the following methods.

That is, the spherical silica composite oxide particulate can be produced by a method (commonly referred to as a sol-gel method) of adding a mixed solution including a hydrolyzable organic silicon compound and an organic compound of a hydrolysable metal (that is a metal component of a composite oxide to be compounded with silica) (also referred to as a "metal to be compounded") to an alkaline solvent in which the organic silicon compound and the organic compound of a metal to be compounded are dissolved but the reaction product remains substantially insoluble to carry out hydrolysis, thereby precipitating the reaction product (see Patent Literatures 1 and 2).

Further, one method of producing an irregularly shaped silica composite oxide particulate includes melting a mixture of silica and a compounded metal oxide at a temperature of the melting point or higher to obtain a glassy material and pulverizing the glassy material. However, it is generally produced by a method of dissolving an alkoxysilane compound in an organic solvent, adding water thereto, carrying out partial hydrolysis, and then, further adding an alkoxide and an alkali metal compound of another metal to be compounded, carrying out hydrolysis to produce an (agar-like) gel-like product, then drying the gel-like product, pulverizing it as necessary, and firing it (see Patent Literatures 1 and 2). Then, as an example of the production of an irregularly shaped silica composite oxide particulate using such a method, Patent Literature 1 describes that a particulate that includes an irregularly shaped inorganic particle formed of a composite oxide of zirconium partially including tetragonal crystals of zirconia (or titanium including a small amount of anatase crystals), silicon, and sodium, which is at least partially amorphous, and has a predetermined average particle diameter was obtained by adding hydrochloric acid to a solution in which tetraethyl silicate is dissolved in isobutanol, carrying out partial hydrolysis, then adding tetrabutyl zirconate (or tetrabutyl titanate) and sodium methylate thereto, stirring the mixture, adding water during the stirring to further promote the hydrolysis, drying the obtained gel, pulverizing it, firing it at 900°C for 2 hours, and then classifying it.

### Citation List

### Patent Literature

Patent Literature 1: Japanese Patent Application Laid-open No. 1990-132102
Patent Literature 2: Japanese Patent No. 4148334
Patent Literature 3: WO 2019-054507

### Non-Patent Literature

Non-Patent Literature 1: ABE Toshihiko, TORII Kazuo, "The determination of amorphous silica and Fe2O3 by X-ray background method", Nendo Kagaku; Journal of the Clay Science Society of Japan, Vol. 14, No. 4, 109-115 (1974)

### Disclosure of Invention

### Technical Problem

In a "dental mill blank that includes a portion to be cut formed of a hybrid resin (hereinafter, also referred to as an "HR blank"), which can also be used to prepare a prosthesis for molars desired to have high strength, it is conceivable that an irregularly shaped silica composite oxide particulate and a spherical silica composite oxide particulate are preferably used in combination as an inorganic filler for the reasons described above. However, as the content ratio of the irregularly shaped particulate increases, the surface smoothness of the cured body tends to decrease. According to the present inventors' research, for example, it has been found that when the content ratio of the irregularly shaped particulate to the total mass of the irregularly shaped silica composite oxide particulate and the spherical silica composite oxide particulate in the inorganic filler to be blended exceeds 50 parts by mass, the polishing properties of the cured body are reduced, and polishing the prosthesis prepared using the HR blank to achieve an aesthetically pleasing gloss requires a considerable amount of time.

In this regard, the present invention aims to provide an irregularly shaped silica composite oxide particulate to be blended in a dental curable composition that includes a polymerizable monomer, an inorganic filler including a spherical silica composite oxide inorganic particulate and an irregularly shaped silica composite oxide particulate, and a polymerization initiator (hereinafter, also referred to as a "spherical-irregularly shaped silica composite oxide filler-containing dental curable composition"), which is capable of providing a cured body with transparency and does not easily reduce the polishing properties of the cured body and is capable of easily achieving an aesthetically pleasing gloss through polishing even when the blending amount thereof is increased to enhance the strength of the cured body, and a method of producing the same, and therefore, an HR blank capable of producing a prosthesis characterized by having transparency and high strength and providing an aesthetically pleasing gloss easily through polishing, and a spherical-irregularly shaped silica composite oxide filler-containing dental curable composition as a raw material thereof.

### Solution to Problem

The present invention is intended to solve the problem described above. A first embodiment of the present invention is an irregularly shaped silica composite oxide particulate that includes a non-porous silica composite oxide particle including silicon and at least one metal, characterized in that
a molar ratio of the at least one metal to a total amount of the silicon and the at least one metal in the silica composite oxide particle is 0.05 or more and 0.25 or less,
the irregularly shaped silica composite oxide particulate has an average particle diameter of 1 µm or more and 10 µm or less, which is defined as a median diameter in a volume-based particle size distribution obtained by a laser diffraction/scattering method,
in a diffraction pattern obtained by measuring X-ray diffraction for the irregularly shaped silica composite oxide particulate, a ratio B/A of diffraction intensity B at a peak top of a peak with the highest diffraction intensity among diffraction peaks derived from each oxide of the at least one metal to diffraction intensity A at a peak top of a halo peak derived from amorphous silica, which appears around 2θ = 22° is 0.5 or more and 0.9 or less.

In the irregularly shaped silica composite oxide particulate according to the above embodiment (hereinafter, also referred to as an "the irregularly shaped silica composite oxide particulate according to the present invention"), it is preferable that a specific surface area measured by a BET method is 3 m²g⁻¹ or more and 8 m²g⁻¹ or less, and in a pore distribution obtained by BJH method measurement, a total pore volume in a range of pore diameters of 10 nm or more and 50 nm or less is 0.06 cm³g⁻¹ or less.

Further, it is preferable that the at least one metal includes at least one of Zr or Ti and optionally Na.

A second embodiment of the present invention is a method of producing an irregularly shaped silica composite oxide particulate that includes a non-porous silica composite oxide particle including silicon and a metal that includes at least one of Zr or Ti (hereinafter, also referred to as an "irregularly shaped silica-zirconia or silica-titania particulate according to the present invention").

This production method includes a hydrolysis step of preparing a silica component raw material liquid that includes a hydrolysate of alkyl silicate and a condensed product of the hydrolysate by hydrolyzing at least part of the alkyl silicate in a reaction solution that includes an aqueous inorganic acid solution and a mixed solution of alkyl silicate and an alcohol, a sol preparation step of preparing a precursor sol in which a silica composite oxide precursor particle that includes silicon and a metal that includes at least one of Zr or Ti is dispersed by mixing a metal raw material that includes at least one of alkyl zirconate and alkyl titanate with the silica component raw material liquid, a gel forming step of forming a precursor wet gel by mixing 100 parts by mass of the precursor sol and 5 parts by mass or more and 10 parts by mass or less of water, a pulverization step of obtaining an irregularly shaped precursor particulate by drying the precursor wet gel and then pulverizing it, and a firing step of producing a non-porous silica composite oxide particle by firing the precursor particulate.

In the hydrolysis step, (a) the alcohol is a branched alkyl alcohol having 3 to 5 carbon atoms, (b) a concentration of protons contained in the aqueous inorganic acid solution relative to the reaction solution is 0.4 (mmol/L) or more and 0.9 (mmol/L) or less, (c) a volume concentration of water in the reaction solution is 2 (vol%) or more and 4 (vol%) or less, (d) a ratio of protons (mmol) contained in the aqueous inorganic acid solution to 1 (mmol) of the alkyl silicate is 0.02 (mol%) or more and 0.04 (mol%) or less, and (e) a ratio of an amount of water (mmol) contained in the reaction solution to 1 (mmol) of the alkyl silicate is 50 (mol%) or more and 100 (mol%) or less. Hydrolysis and a condensation reaction are carried out using the reaction solution.

When, in a gas chromatogram obtained by performing gas chromatography analysis on the silica component raw material liquid prepared in the hydrolysis step, a peak area of a peak attributed to an unhydrolyzed product (a1) of alkyl silicate having no hydroxy group, which has at least some functional groups that may be substituted with alkoxy groups by the alcohol in the mixed solution, is defined as S₁, a peak area of a hydroxysilicate monomer (a2) that is a hydrolysate of the (a1), which has at least one hydroxy group and contains one silicon atom, is defined as S₂, and a peak area of a silicate dimer (a3) that is a condensed product of the (a2) and/or a condensed product of the (a1) and the (a2), which contains two silicon atoms, is defined as S₃, a peak area ratio of S₂ to S₁ is 0.3 or more and 0.6 or less and a peak area ratio S₃ to S₁ is 0.03 or more and 0.5 or less,

In the sol preparation step, the metal raw material and the silica component raw material liquid are mixed such that a molar ratio of the metal to a total amount of the silicon and the metal included in the silica composite oxide precursor particle is 0.05 or more and 0.25 or less and a content ratio of the silicon and the metal included in the silica composite oxide precursor particle to the precursor sol is 1.5 (mol/L) or more and 3 (mol/L) or less.

In the firing step, the firing is performed within a range of 700°C or more and 1000°C or less.

The metal raw material may include a sodium alkoxide.

A third embodiment of the present invention is a dental curable composition, comprising:
a polymerizable monomer: 100 parts by mass;
a spherical inorganic particulate having an average particle diameter of 0.1 µm or more and 5 µm or less: 100 parts by mass or more and 200 parts by mass or less;
a spherical inorganic particulate having an average particle diameter of 0.01 µm or more and 0.1 µm or less: 40 parts by mass or more and 100 parts by mass or less;
the irregularly shaped silica composite oxide particulate according to the present invention: 100 parts by mass or more and 250 parts by mass or less; and
a polymerization initiator: 0.1 parts by mass or more and 1 part by mass or less (hereinafter, also referred to as a "dental curable composition according to the present invention").

A fourth embodiment of the present invention is a dental blank for cutting that includes a to-be-cut portion, characterized in that at least part of the to-be-cut portion is formed of a cured body of the dental curable composition according to the present invention (hereinafter, also referred to as a "blank according to the present invention").

### Advantageous Effects of Invention

The irregularly shaped silica composite oxide particulate according to the present invention is characterized in that when used as an irregularly shaped silica composite oxide particulate component of the spherical-irregularly shaped silica composite oxide filler-containing dental curable composition, it does not easily reduce the polishing properties of the cured body even when the blending amount thereof is increased to enhance the strength of the cured body and provides an aesthetically pleasing gloss easily through polishing. Further, the production method according to the present invention allows the irregularly shaped silica composite oxide particulate according to the present invention having the above excellent characteristics to be produced efficiently.

### Brief Description of Drawings

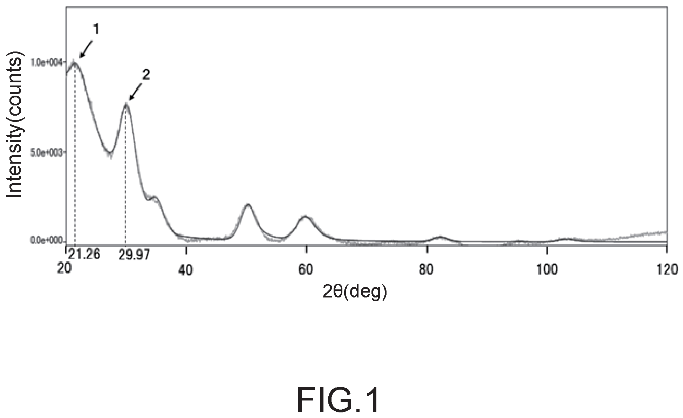

[Fig. 1] Fig. 1 shows a diffraction pattern obtained by measuring X-ray diffraction for the irregularly shaped silica composite oxide particulate according to the present invention in Example 1. Mode(s) for Carrying Out the Invention

It is conceivable that a porous irregularly shaped silica composite oxide particulate obtained by a method such as obtaining a wet silica composite oxide using a wet method such as a so-called sol-gel method and then firing it at a low temperature can be easily polished, and therefore, using such an irregularly shaped particulate allows the polishing properties of the cured body of the spherical-irregularly shaped silica composite oxide filler-containing dental curable composition to be improved. However, in such a porous irregularly shaped silica composite oxide particulate, gaps can serve as a fracture origin, resulting in reduced strength when used as a prosthesis formed of the cured body or its molded body in some cases. Further, when a pulverized product of ceramics with low toughness such as silica is used instead of the irregularly shaped silica composite oxide particulate, the refractive index difference from the resin component in the cured body increases and light scattering becomes significant, impairing transparency.

The present inventors hypothesized that the polishing properties of the non-porous (solid) irregularly shaped silica composite oxide particulate (specifically, the cured body of the spherical-irregularly shaped silica composite oxide filler-containing dental curable composition including this) are influenced by the microstructure and crystallinity of the composite oxide forming each particle, examined the microstructure and crystallinity of the above silica-zirconia irregularly shaped particulate disclosed in Patent Literature 1, which is an existing irregularly shaped silica composite oxide particulate with low polishing properties, prepared irregularly shaped silica composite oxide particulates by varying the production conditions, and investigated the microstructure, crystallinity, and polishing properties of the numerous irregularly shaped silica composite oxide particulates obtained.

As a result, they could not find a clear difference in the microstructure due to analytical limitations, but found that the composite oxide in the existing irregularly shaped silica composite oxide particulate exhibits relatively high crystallinity (see Comparative Example 8) and that when a specific condition is adopted, an irregularly shaped silica composite oxide particulate formed of a composite oxide with significantly lower crystallinity (significantly higher amorphousness) than the above composite oxide can be obtained and using this is less likely to impair the polishing properties (of the cured body of the spherical-irregularly shaped silica composite oxide filler-containing dental curable composition) even when the blending amount thereof is increased, leading to the completion of the present invention.

Each embodiment of the present invention will be described below in detail. Note that in the present specification, the notation "x to y" using numerical values x and y means "x or more and y or less", unless otherwise specified. In such a notation, in the case where a unit is attached to only the numerical value y, the unit applies also to the numerical value x. Further, in this specification, the term "(meth)acrylic" means both "acrylic" and "methacrylic". Similarly, the term "(meth)acrylate" means both "acrylate" and "methacrylate", and the term "(meth)acryloyl" means both "acryloyl" and "methacryloyl".

### 1. Irregularly shaped silica composite oxide particulate according to present invention

The irregularly shaped silica composite oxide particulate according to the present invention is an irregularly shaped particulate that includes a non-porous silica composite oxide particle including silicon and at least one metal. Here, the irregularly shaped particulate is a particulate in which the individual particles forming the particulate are non-spherical (non-true spherical) and the outer shapes of the individual particles are irregular.

Here, "non-porous" refers to solid particles that have no or substantially no pores communicating with the outside. Specifically, it means that the specific surface area measured by a BET method is 3 to 8 m²g⁻¹, and in the pore distribution obtained by BJH method measurement, the total pore volume in a range of pore diameters of 10 to 50 nm is 0.06 cm³g⁻¹ or less. If the specific surface area exceeds 8 m²g⁻¹ or the pore volume exceeds 0.06 cm³g⁻¹, there is a possibility that the densification of the silica composite oxide particle has not progressed sufficiently and gaps communicating with the outside are left.

Further, the irregularly shaped silica composite oxide particulate according to the present invention needs to satisfy the conditions shown in the following (1), (2), and (3).
(1) In the silica composite oxide particle, the molar ratio of at least one metal to the total amount of silicon and at least one metal is 0.05 or more and 0.25 or less.
   That is, when the total amount (mol) of silicon atoms contained in the composite oxide that is the material of a silica composite oxide particle is defined as [Si] and the total amount (mol) of the at least one metal contained in the above composite oxide is defined as [M], the ratio of [M] to [Si] and [M]: [M]/([Si]+[M]) is 0.05 to 0.25.
(2) The above irregularly shaped silica composite oxide particulate has an average particle diameter of 1 to 10 µm, which is defined as a median diameter in the volume-based particle size distribution obtained by a laser diffraction/scattering method.
(3) In the diffraction pattern obtained by measuring X-ray diffraction for the above irregularly shaped silica composite oxide particulate, the ratio B/A of a diffraction intensity B at a peak top of a peak with the highest diffraction intensity among diffraction peaks derived from each oxide of the at least one metal to diffraction intensity A at a peak top of a halo peak derived from amorphous silica, which appears around a diffraction angle 2θ = 22° is 0.5 to 0.9.

The silica composite oxide that is a material of the above non-porous silica composite oxide particle forming the irregularly shaped silica composite oxide particulate according to the present invention is typically a metal oxide having no absorption band due to d-d transitions in the visible light wavelength range, and at least one metal selected from the group consisting of Group 2, Group 3, and Group 4 elements of the periodic table is used as the at least one metal that is a metal in the metal oxide. That is, magnesium (Mg), calcium (Ca), strontium (Sr), and barium (Ba) can be used as the metal element in the Group 2 of the periodic table, scandium (Sc), yttrium (Y), and lanthanoids can be used as the metal element in the Group 3 of the periodic table, and titanium (Ti), zirconium (Zr), and hafnium (Hf) can be used as the metal element in the Group 4 of the periodic table. Of these metal elements, at least one metal element selected from the group consisting of Ba, Ti, and Zr is preferable, and Zr or Ti is particularly preferable. These metals form, as oxides corresponding to their valence, e.g., as oxides such as BaO, TiO₂, and ZrO₂, a composite oxide with a silicon oxide (silica: SiO₂). That is, in the above particularly preferable aspect, silica-zirconia or silica-titania is obtained.

Further, the silica composite oxide may include Na₂O for the purpose of suppressing the influence of strong acid sites on the surface and improving the effect of densification by firing. In this case, the at least one metal includes Na in addition to the above metal.

As shown in the above (1), the above composite oxide needs to have a composition that [M]/([Si]+[M]) is 0.05 to 0.25. By having such a composition, it becomes possible to adjust the refractive index of the composite oxide particle to 1.48 to 1.56, obtain a value that is the same or close to the refractive index of the cured body of the polymerizable monomer commonly used in the dental curable composition, and make the cured body of the spherical-irregularly shaped silica composite oxide filler-containing dental curable composition have transparency. For the reasons of imparting high X-ray contrast properties and improving the polishing properties of the cured body of the dental curable composition, [M]/([Si]+[M]) is preferably 0.05 to 0.2, particularly favorably 0.05 to 0.15.

Further, as shown in the above (2), the average particle diameter defined as a median diameter in the volume-based particle size distribution obtained by a laser diffraction/scattering method needs to be 1 to 10 µm. When the above average particle diameter is less than 1 µm, the dilatancy of the dental curable composition increases, resulting in reduced operability. When the average particle diameter exceeds 10 µm, the strength of the cured body of the dental curable composition is reduced. From the viewpoints of achieving both strength and operability, the above average particle diameter is preferably 0.05 to 50 µm, particularly preferably 0.1 to 10 µm.

Further, as shown in the above (3), the diffraction pattern obtained by measuring X-ray diffraction for the irregularly shaped silica composite oxide particulate according to the present invention needs to satisfy the condition that B/A = 0.5 to 0.9.

Here, as a method of measuring X-ray diffraction, for example, a powder X-ray diffraction measurement method performed under conditions of X-ray output: 40 kV, 30 mA, a scan axis: 2θ/θ, and a scan range: 5 deg to 120 deg can be adopted. Note that amorphous silica is known to have a halo peak derived from amorphous silica appearing around a diffraction angle: 2θ = 25° (see Non-Patent Literature 1), and in the X-ray diffraction pattern for the irregularly shaped silica composite oxide particulate, this halo peak and the diffraction peak derived from each oxide of the at least one metal are observed to overlap with each other. The A in the condition (3) refers to the diffraction intensity (cps) at the peak top of the above halo peak (peak 1 appearing around 2θ = 22° in Fig. 1), and the B refers to the diffraction intensity (cps) at the peak top of a peak with the highest diffraction intensity among diffraction peaks derived from each oxide of the at least one metal, e.g., a peak (peak 2 in Fig. 1) appearing around a diffraction angle: 2θ = 30° in the case of zirconia. Further, in the case of titania, it refers to a peak appearing around a diffraction angle: 2θ = 25°.

When the B/A is less than 0.5, the bending strength of the cured body of the spherical-irregularly shaped silica composite oxide filler-containing dental curable composition tends to be reduced. When the B/A exceeds 0.9, the transparency of the above cured body is significantly reduced. Note that the mechanism of this opacification is not necessarily clear. However, it is conceivable that light scattering is occurring at the silica-metal oxide interface due to the selective crystallization of the silica component and metal oxide component of the silica composite oxide. The B/A is preferably 0.50 to 0.85, particularly preferably 0.50 to 0.80.

### 2. Method of producing present invention

The production method according to the present invention is a method of producing the irregularly shaped silica composite oxide particulate according to the present invention in which the at least one metal includes at least one of Zr or Ti (i.e., the irregularly shaped silica-zirconia or silica-titania particulate according to the present invention), and includes, similarly to the method of producing a non-porous irregularly shaped silica-zirconia particulate and a non-porous irregularly shaped silica-titania particulate described in Patent Literature 1 or 2, a hydrolysis step; a sol preparation step; a gel forming step; a pulverization step; and a firing step described below. Note that the at least one metal may optionally include Na.

Hydrolysis step: a step of preparing a silica component raw material liquid that includes a hydrolysate of alkyl silicate and a condensed product of the hydrolysate by using alkyl silicate as a silicon source, adding an aqueous inorganic acid solution to a mixed solution of the alkyl silicate and an alcohol, and hydrolyzing at least part of the alkyl silicate.

In the above hydrolysis step, a silica component raw material liquid that includes a hydrolysate of alkyl silicate and a condensed product of the hydrolysate is prepared by hydrolyzing at least part of alkyl silicate in a reaction solution that includes an aqueous inorganic acid solution and a mixed solution of alkyl silicate and an alcohol under specific conditions described below. The silica component raw material liquid prepared in this way does not precipitate solid particles. Further, in a gas chromatogram obtained by performing gas chromatography analysis on the silica component raw material liquid, a specific peak area ratio for the unhydrolyzed product, hydrolysate, and condensate as described below, can be obtained.

Sol preparation step: a step of preparing a silica composite oxide precursor sol (also referred to simply as a "precursor sol") by using a metal raw material that includes at least one of alkyl zirconate or alkyl titanate as a metal source of the at least one metal and mixing the silica component raw material liquid that includes Si in an amount corresponding to the above [Si] with the above metal raw material in an amount corresponding to [M] where the above [M]/([Si]+[M]) is 0.05 to 0.25.

In the above sol preparation step, the metal raw material and the silica component raw material liquid are mixed such that the molar ratio of silicon to the total amount of silicon and metal included in the silica composite oxide precursor particle is 0.05 to 0.25. In this way, a precursor sol in which a silica composite oxide precursor particle that includes silicon and at least one metal of Zr or Ti at the above molar ratio is dispersed is prepared. Further, in the silica composite oxide particle finally produced in this way, the above [M]/([Si]+[M]) is 0.05 to 0.25.

Gel forming step: a step of forming a silica composite oxide precursor wet gel (also referred to simply as a "precursor wet gel") by mixing 100 parts by mass of the above silica composite oxide precursor sol and 5 to 10 parts by mass of water.

Pulverization step: a step of obtaining an irregularly shaped silica composite oxide precursor particulate (also referred to simply as a "precursor particulate") by drying the above silica composite oxide precursor wet gel and then pulverizing it.

Firing step: a step of firing the above silica composite oxide precursor particulate.

In the above firing step, a non-porous silica composite oxide particle is produced by firing a precursor particulate that includes a silica composite oxide precursor particle at a specific temperature described below.

Then, the production method according to the present invention allows the irregularly shaped silica-zirconia or silica-titania particulate according to the present invention to be produced efficiently by performing the above hydrolysis step, the above sol preparation step, and the above firing step under specific conditions.

Further, it is obvious from the conditions of the sol preparation step that the above irregularly shaped silica composite oxide particulate obtained by the production method according to the present invention satisfies the above condition (1) that the irregularly shaped silica composite oxide particulate according to the present invention should satisfy. Further, the above condition (2) can also be satisfied easily by performing an operation such as classification as necessary after the pulverization step or the firing step. Further, the above condition (3) can be satisfied by performing the above hydrolysis step and gel forming step such that the above conditions are satisfied.

Each step of the production method according to the present invention will be described below in detail.

### 2-1. Hydrolysis step

In the hydrolysis step, alkyl silicate is used as a silicon source, an aqueous inorganic acid solution is added to the mixed solution of alkyl silicate and an alcohol, and at least part of the alkyl silicate is hydrolyzed to prepare the above silica component raw material liquid that includes a hydrolysate of alkyl silicate and a condensed product of the hydrolysate. At this time, in the production method according to the present invention, the hydrolysis and the condensation reaction are carried out such that the following conditions (a) to (e) are satisfied, thereby preparing a silica component raw material liquid having a specific composition that satisfies the following condition (f) without precipitating solid particles.
(a) A branched alkyl alcohol having 3 to 5 carbon atoms (hereinafter, also referred to as a "specific alcohol") is used as the above alcohol.
(b) The concentration of protons contained in the aqueous inorganic acid solution relative to the above reaction solution (i.e., the proton concentration obtained by dividing the amount of protons contained in the above aqueous inorganic acid solution by the total volume (L) of the reaction solution): [H⁺] is 0.4 to 0.9 (mmol/L).
(c) The volume concentration of water in the reaction solution (i.e., the volume concentration of water obtained by dividing the amount of water (L) contained during hydrolysis by the total volume of the reaction solution) is 2 to 4 (vol%).
(d) The ratio of protons (mmol) contained in the above aqueous inorganic acid solution relative to 1 (mmol) of alkyl silicate (i.e., the amount of protons(mmol) relative to 1 (mmol) of alkyl silicate): {([H⁺]/[Si])×100} is 0.02 to 0.04 (mol%).
(e) The ratio of the amount of water (mmol) contained in the reaction solution relative to 1 (mmol) of alkyl silicate (i.e., the above amount of water (mmol) relative to 1 (mmol) of the above alkyl silicate): {([H₂O]/[Si])×100} is 50 to 100 (mol%).
(f) When, in a gas chromatogram obtained by performing gas chromatography analysis on the silica component raw material liquid prepared under the above conditions, a peak area of a peak attributed to an unhydrolyzed product (a1) of alkyl silicate having no hydroxy group, which has at least some functional groups that may be substituted with alkoxy groups by the alcohol in the mixed solution, is defined as S₁, a peak area of a hydroxysilicate monomer (a2) that is a hydrolysate of the (a1), which has at least one hydroxy group and contains one silicon atom, is defined as S₂, and a peak area of a silicate dimer (a3) that is a condensed product of the (a2) and/or a condensed product of the (a1) and the (a2), which contains two silicon atoms, is defined as S₃, a peak area ratio of S₂ to S₁ is 0.3 to 0.6 and a peak area ratio S₃ to S₁ is 0.03 to 0.5. Note that in the above condensed product of the (a2), two (a2) may be the same or different from each other. The above alkoxy group is derived from the alcohol (solvent) in the mixed solution.

In other words, the above (a1) is an unhydrolyzed product of those having no hydroxy group, of unreacted alkyl silicate and alkyl silicate having at least one functional group substituted with a solvent alcohol. The above (a2) is a hydroxysilicate monomer formed of a compound containing one silicon atom, in which unreacted alkyl silicate and alkyl silicate having at least one functional group substituted with a solvent alcohol have at least one alkoxy group substituted with a hydroxy group. The above (a3) is a silicate dimer formed of a compound containing two silicon atoms, in which the hydroxysilicate monomer (a2) is condensed with another hydroxysilicate monomer (a2) or alkyl silicate used as a raw material to form a siloxane bond.

Note that regarding the above (f), as the gas chromatography analysis, gas chromatography analysis in which the stationary phase contains dimethylpolysiloxane, the mobile phase is helium, and the detector is a flame ionization detector (FID) is suitably adopted. Further, the identification of the following peaks derived from (a1) to (a3) can be performed by GC-MS analysis.

By using such a silica component raw material liquid and performing the firing in the firing step at temperatures within the range of 700 to 1000°C as described below, the crystallinity specified in the above condition (3) is provided. The reason for this is not necessarily clear, but it is presumed as follows. That is, by making the alkyl silicate concentration and the amount of water relative to alkyl silicate relatively high and the proton concentration relatively low and carrying out hydrolysis and the accompanying condensation reaction in a reaction solution using a branched alkyl alcohol having 3 to 5 carbon atoms (specific alcohol), an alkoxide bonded to the silicon atom in the raw material silicate undergoes a transesterification reaction with the specific alcohol, thereby obtaining silicate to which the alkoxide derived from the specific alcohol is bonded. It is conceivable that the alkoxide derived from the specific alcohol is bulky and thus is less susceptible to a hydrolysis reaction as compared with the raw material silicate. For this reason, the condensation reaction forming these oligomers proceeds mildly, and a silica component raw material liquid that includes the hydroxysilicate monomer (a2) and the silicate dimer (a3) at relatively high concentrations can be prepared without generating a complex condensate that precipitates as a solid. Then, it is conceivable that in the sol preparation step, the formation of bonds between metal atoms via an oxygen atom (M-O-M) is suppressed, which makes it easier to form a metallosiloxane bond (Si-O-M; M is a metal atom), and a complex in which a metal atom is incorporated into the silicate oligomer is condensed to form a silica composite oxide precursor sol. For this reason, it is conceivable that in the firing step, the selective crystallization of an oxide of the metal atom is less likely to occur and amorphous silica disrupts the periodic arrangement of the metal elements, enhancing the amorphous properties.

From the viewpoint of effect, [H⁺] is preferably 0.45 to 0.85 (mmol/L), and 100 × [H₂O] / [Si] is preferably 60 to 90 (mol%). Further, S₂/S₁ is preferably 0.3 to 0.55, and S₃/S₁ is preferably 0.03 to 0.2.

As alkyl silicate to be used as a silicon source, ethyl silicate, methyl silicate, butyl silicate, or the like can be used.

As the branched alkyl alcohol having 3 to 5 carbon atoms (specific alcohol), an alcohol such as 2-propanol, 2-methyl-1-propanol, 2-butanol, and 2-methyl-1-butanol can be suitably used. In this step, at least one type of specific alcohol only needs to be included, and a plurality of types of specific alcohols may be mixed and used.

As the aqueous inorganic acid solution used in this step, an aqueous solution using an inorganic acid can be used without any restrictions. However, a hydrochloric acid aqueous solution, a nitric acid aqueous solution, and a sulfuric acid aqueous solution are preferably used due to their industrial availability.

The temperature at which hydrolysis is carried out only needs to be a temperature at which various raw materials are miscible in the liquid state, and is preferably 30 to 50°C.

### 2-2. Sol preparation step

In the sol preparation step, a metal raw material that includes at least one of alkyl zirconate or alkyl titanate and a sodium alkoxide optionally is used as the metal source of the at least one metal, and the above silica component raw material liquid including Si in an amount corresponding to the above [Si], the above metal raw material in an amount corresponding to [M] where the above [M]/([Si]+[M]) is 0.05 to 0.25, and a base as appropriate are mixed in accordance with the composition of the target irregularly shaped silica-zirconia or silica-titania particulate according to the present invention, thereby preparing a silica composite oxide precursor sol.

As alkyl zirconate to be used as the above metal raw material, tetrapropyl zirconate, tetrabutyl zirconate, or the like can be used. However, tetra-n-butyl zirconate is particularly preferably used because it is more stable, easier to handle, and less expensive. Further, as alkyl titanate, tetrapropyl titanate, tetrabutyl titanate, or the like can be used. However, tetraisopropyl titanate is particularly preferably used because it is inexpensive.

A sodium alkoxide to be used as the above metal raw material is used as necessary to adjust the acidity of the composite oxide particulate and prevent discoloration/coloration and a decrease in strength of the obtained cured body. As the sodium alkoxide, sodium methoxide and/or sodium ethoxide is suitably used. The sodium alkoxide does not necessarily need to be used. However, in the case where a sodium alkoxide is used, it is used in an amount that [Na] / ([Si] + [Zr] + [Na]) or [Na] / ([Si] + [Ti] + [Na]) will be 0.01 to 0.1, particularly 0.02 to 0.05. Note that since the sodium alkoxide functions as a base by generating OH⁻ in coexistence with water, the (a1) to (a3) components included in the silica component raw material liquid and the metal raw material are (hydrolyzed/dehydrated) condensed to form a silica composite oxide precursor sol in which a silica composite oxide precursor particle is dispersed in the dispersion medium (also referred to simply as a "precursor sol").

The mixing of the above silica component raw material liquid and the above metal raw material can be suitably performed by stirring with a stir bar and a stirrer at room temperature of approximately 15 to 45°C.

In the production method according to the present invention, in the sol preparation step, the content ratio of the silica composite oxide precursor particle in the silica composite oxide precursor sol needs to be 1.5 to 3.0 (mol/L) in order to obtain a uniform gel efficiently. That is, the content ratio of the silica composite oxide precursor particle calculated by dividing the sum of the total amount (mol) of silicon and metal contained in the silica composite oxide by the volume (L) of the silica composite oxide precursor sol is adjusted to 1.5 to 3 (mol/L). Here, the silica composite oxide precursor particle refers to each inorganic component included in silica and the composite oxide raw material, and their number of moles can be determined on the basis of the added amount of the raw material, the concentration, and the molecular weight of each component. When the above content ratio is less than 1.5 (mol/L), the gelling time becomes significantly longer and the productivity is reduced, which is not preferable. Further, when the above content ratio exceeds 3.0 (mol/L), a uniform agar-like gel cannot be obtained, and an opaque and glass-like solid precipitates unevenly from the sol, which is not preferable. Note that the adjustment of the above content ratio can be performed by adjusting the added amount of the raw material, adding a solvent or water, or removing a solvent or water through drying.

### 2-3. Gel forming step

This step is a step of mixing 5 to 10 parts by mass of water to 100 parts by mass of the above silica composite oxide precursor sol to form the silica composite oxide precursor wet gel. Mixing the silica composite oxide precursor sol and water causes the hydrolysis and condensation of the dispersoid in the sol to proceed, and the molecular structures become entangled, increasing the viscosity to provide a solid gel that incorporates the dispersion medium and water.

From the viewpoint of obtaining a uniform gel, when mixing the silica composite oxide precursor sol and water, it is preferable to perform stirring with a stir bar and a stirrer in order to prevent localized gelling due to uneven concentration. When the amount of water is less than 5 parts by mass, gelling does not occur, which is not preferable. When the amount of water exceeds 10 parts by mass, no gel is obtained due to precipitation and settlement of solid silica particles, which is not preferable.

### 2-4. Pulverization step

This step is a step of collecting the wet gel formed in the previous step, drying it to obtain a dried gel, and roughly adjusting the particle size. As the method of drying the wet gel, a known drying method can be used without any restrictions. However, from the viewpoint of obtaining a more dense silica composite oxide particle, it is preferable to set the residual ratio of the incorporated dispersion medium and water to 40 mass% or less, and the drying conditions are a drying temperature between 80 and 150°C and a drying time of 1 to 18 hours because a gel with such a residual ratio can be easily obtained.

As a pulverization method, for example, ball mill pulverization using a pot and a ball formed of yttria-reinforced zirconia can be suitably used. Pulverization before firing allows pulverization and particle size control after the firing step to be performed easily.

Since the average particle diameter of the irregularly shaped silica-zirconia or silica-titania particulate according to the present invention that is a target product is 1 to 10 µm, pulverization only needs to be performed such that an average particle diameter larger than the target average particle diameter within the above range is obtained. However, it is preferable to perform pulverization such that an average particle diameter approximately 100% larger than the target average particle diameter within the above range is obtained. Note that after the pulverization, for example, classification with a sieve or elutriation classification may be performed as necessary.

### 2-5. Firing step

In this step, the powder obtained in the preceding step is fired. As the firing method, a known method can be used without any restrictions. The firing temperature is 700 to 1000°C, favorably 800 to 950°C from the viewpoint of removing the liquid components to obtain a particulate having B/A of 0.5 to 0.9 and the viewpoint of preventing fusion between particles.

### 2-6. Other steps

As another step, a further classification operation may be performed to narrow the particle size distribution. Further, powder particles of the obtained silica composite oxide particle may be subjected to silane coupling treatment in order to improve the affinity with the organic matrix when blending the powder particles in the dental composition. As the silane coupling agent used in the silane coupling treatment, any known silane coupling agent can be used without any restrictions.

### 3. Dental curable composition according to the present invention and blank according to the present invention

The dental curable composition according to the present invention includes a polymerizable monomer: 100 parts by mass, a spherical inorganic particulate having an average particle diameter of 0.1 to 5 µm: 100 to 200 parts by mass (favorably 100 to 150 parts by mass), a spherical inorganic particulate having an average particle diameter of 0.01 to 0.1 µm: 40 to 100 parts by mass (favorably 40 to 80 parts by mass), the irregularly shaped silica composite oxide particulate according to the present invention: 100 to 250 parts by mass (favorably 150 to 250 parts by mass), and a polymerization initiator: 0.1 to 1 parts by mass.

The dental curable composition according to the present invention corresponds to a photocurable dental restoration material that includes a mixture of a spherical inorganic particulate having an average particle diameter of 0.1 to 5 µm and a spherical inorganic particulate having an average particle diameter of 0.01 to 0.1 µm, and an irregularly shaped inorganic particulate having an average particle diameter of 1 to 9 µm, which is disclosed in Patent Literature 2, in which the irregularly shaped silica composite oxide particulate according to the present invention is used instead of the irregularly shaped inorganic particulate and the blending amount of each particulate is within the above range. Therefore, as each component other than the irregularly shaped silica composite oxide particulate according to the present invention, those disclosed in Patent Literature 2 as being usable in the above photocurable dental restoration material can be used without particular restrictions. However, the dental curable composition according to the present invention does not necessarily need to be used as a photocurable dental restoration material, and can also be used suitably as a raw material composition of a so-called hybrid resin, which forms a to-be-cut portion of a dental blank for cutting.

Each component other than the irregularly shaped silica composite oxide particulate according to the present invention in the dental curable composition according to the present invention will be described briefly. As a polymerizable monomer, 2,2-bis(4-methacryloyloxypolyethoxyphenyl)propane, 1,6-bis(methacrylethyloxycarbonylamino)-2,2-4-trimethylhexane, neopentyl glycol dimethacrylate, triethylene glycol dimethacrylate, ethylene glycol dimethacrylate, diethylene glycol dimethacrylate, butylene glycol dimethacrylate, or the like can be suitably used.

Further, as a spherical inorganic particulate, any material generally used suitably as a spherical inorganic particulate can be used without any restrictions as long as its average particle diameter is within the above range. Specifically, a composite inorganic oxide such as silica-zirconia, silica-titania, and silica-alumina, borosilicate glass, soda glass, glass including a heavy metal (e.g., barium, strontium, zirconium), aluminosilicate, fluoroaluminosilicate, glass ceramics, silica, or the like can be suitably used.

Further, as a polymerization initiator, not only a photopolymerization initiator such as bisacylphosphine oxide disclosed in Patent Literature 2 but also a thermal polymerization initiator such as benzoyl peroxide, p-chlorobenzoyl peroxide, tert-butylperoxy-2-ethylhexanoate, and tert-butyl peroxydicarbonate can be suitably used. Note that in the case where the dental curable composition according to the present invention is used as a raw material composition of a hybrid resin, which forms a to-be-cut portion, it is preferable to use, as a polymerization catalyst, a thermal polymerization catalyst because a toned cured body having a large thickness needs to be obtained when used as a dental resin material for cutting and machining, making it difficult for light to transmit into the interior of the curable composition in some cases.

The dental curable composition according to the present invention can be obtained by sufficiently kneading predetermined amounts of the above essential components and respective optional components added as necessary, and degassing the obtained paste to remove bubbles.

As described above, the dental curable composition according to the present invention is suitable as a raw material composition of a so-called hybrid resin, which forms a to-be-cut portion of a dental blank for cutting, and the blank according to the present invention, which includes a cured body thereof, has the characteristic of having high strength and being capable of providing an aesthetically pleasing gloss easily through polishing. When producing the blank according to the present invention using the dental curable composition according to the present invention, a casting polymerization method using a mold is suitably adopted as described in, for example, Japanese Patent Application Laid-open No. 2019-178105.

### (Example)

Although the present invention will be specifically described below by way of Examples, the present invention is not limited to these Examples.

### 1. Production and evaluation of irregularly shaped silica composite oxide particulate

The abbreviations for compounds used in the production of a composite oxide particle are shown below.
<Silicon source (alkyl silicate)>
   ·TES: tetraethyl silicate (manufactured by Colcoat Co,.Ltd.)
<Metal raw material>
   ·TBZ: 87 mass% tetra(n-butoxy)zirconate n-butyl alcohol solution (manufactured by HOKKO CHEMICAL INDUSTRY CO., LTD.)
   ·TPT: tetraisopropyl titanate (manufactured by Tokyo Chemical Industry Co., Ltd.)

   ·NaOMe: 28 mass% sodium methoxide methanol solution (manufactured by FUJIFILM Wako Pure Chemical Corporation)

### Example 1

### [Hydrolysis step]

A 0.11 mass% aqueous sulfuric acid solution: 60 g and TES: 1056 g were dissolved in 2-methyl-1-propanol (isobutyl alcohol): 0.9 L and mixed at room temperature for 2 hours and 30 minutes to hydrolyze the mixture, thereby preparing a silica component raw material liquid. At this time, the proton concentration obtained by dividing the amount of protons contained in the above aqueous sulfuric acid solution by the total volume (L) of the reaction solution: [H⁺] (also referred to as a "proton concentration relative to the reaction solution") is 0.63 (mmol/L), the volume concentration of water obtained by dividing the amount of water (L) contained during hydrolysis by the total volume of the reaction solution (also referred to as a "water concentration relative to the reaction solution") is 2.8 (vol%), the above amount of protons (mmol) relative to TES: 1 (mmol), which is used as the "amount of protons relative to alkyl silicate": 100 × [H⁺] / [Si] is 0.026 (mol%), and the amount of water (mmol) relative to TES: 1 (mmol), which is used as the "amount of water relative to alkyl silicate": 100 × [H₂O] / [Si] is 64.6 (mol%).

For reference, when the above proton concentration relative to the reaction solution: [H⁺] and water concentration relative to the reaction solution are converted to the volume (L)-based concentration of the silica composite oxide precursor sol obtained in a sol preparation step described below, [H⁺] = 0.53 (mmol/L) and the water concentration = 2.4 (vol%).

GC-MS analysis was performed under the following conditions on the silica component raw material liquid obtained in this way to identify each of the observed peaks, they were classified into an unhydrolyzed product (a1), a hydroxysilicate monomer (a2), and a silicate dimer (a3), a peak area: S1 of a peak attributed to (a1), a peak area: S2 of the above (a2), and a peak area: S3 of the above (a3) were obtained from the sum of strengths of the classified peaks, and S₂/S₁ and S₃/S₁ were further obtained. The results are shown in Table 2. Note that the values of S₂/S₁ and S₃/S₁ are expressed as % (values multiplied by 100) in Table 2.

Identification results of analysis conditions and peaks
·Device: 7890A (manufactured by Agilent Technologies), Column: DB-5
·Initial temperature: 60°C Retention time: 1 min Heating rate: 5°C/min
·Final temperature: 260°C Retention time: 4 min Injection port temperature: 260°C
·Sample injection amount: 1 µL Split ratio: 20:1
·classifications and retention times (rt, unit: min.) of detections peaks attributed thereto are shown below.
   Classification: rt (min.) of each peak
   (a1): 7.7, 11.4, 14.5, 17.6, 20.5 (Total: 5 peaks)
   (a2): 8.5, 12.3, 15.7, 18.7 (Total: 4 peaks)
   (a3): 20.3, 23.0, 25.3, 27.2 (Total: 4 peaks).

### [Sol preparation step]

Subsequently, TBZ: 190 g and NaOMe: 50 g were added to a silica component raw material liquid and stirred for 10 minutes to obtain a silica composite oxide precursor sol. Note that [M] / ([Si] + [M]) is 0.12. Note that the content ratio of a silica composite oxide precursor calculated by dividing the sum of the total amount (mol) of silicon atoms and the total amount (mol) of the at least one metal included in the above composite oxide by the volume (L) of the above silica composite oxide precursor sol (also referred to as a "precursor content ratio") is 2.3 (mol/L).

### [Gel preparation step]

Then, water: 150 g (7 parts by mass with respect to 100 parts by mass of the above silica composite oxide precursor sol) was added to the above silica composite oxide precursor sol while stirring to obtain a silica composite oxide precursor wet gel.

### [Pulverization step and firing step]

The obtained wet gel was allowed to stand at room temperature for 4 hours. After confirming the solidification, it was collected and air-dried at 120°C for 15 hours to dry and solidify. The dried and solidified gel was pulverized with a vibrating ball mill such that the average particle diameter would be 4 µm, thereby obtaining a white powder. After that, the powder was fired at 900°C for 4 hours to obtain an irregularly shaped silica composite oxide particulate F1 corresponding to the irregularly shaped silica composite oxide particulate according to the present invention.

Measurement of powder X-ray diffraction was performed on the obtained F1 to evaluate crystallinity, specifically, to obtain a ratio B/A of a main peak strength: B of ZrO₂ appearing at a diffraction angle (2θ) of 30° to a halo peak strength: A of amorphous silica appearing at a diffraction angle (2θ) of 22°. In addition, various types of analysis were performed to obtain an average particle diameter, a specific surface area, a pore volume, and the like. The obtained X-ray diffraction pattern is shown in Fig. 1, and the various analysis results are shown in Table 2. Note that the various types of analysis were performed as follows.

### (1) Measurement of powder X-ray diffraction

Measurement of powder X-ray diffraction for evaluating the crystallinity of an irregularly shaped silica composite oxide particulate was performed using an X-ray diffractometer (manufactured by Rigaku Holdings Corporation and its Global Subsidiaries) with X-ray output: 40 kV, 30 mA, a detector: D/teX Ultra, a scan axis: 2θ/θ, and a scan range: 5 deg to 120 deg.

### (2) Measurement of average particle diameter

Measurement of the particle size distribution for evaluating the average particle diameter of an irregularly shaped silica composite oxide particulate was performed using a particle size distribution measuring apparatus (Beckman Coulter, Inc.) with a dispersoid refractive index: 1.36, a dispersion medium refractive index: 1.55, and a dispersion solvent: ethanol.

### (3) Measurement of specific surface area and pore volume

Measurement of the specific surface area and pore volume of an irregularly shaped silica composite oxide particulate was performed by measuring nitrogen adsorption at 77 K (kelvin) using a specific surface area/pore distribution measuring apparatus (manufactured by Microtrac MRB).

### (4) Measurement of refractive index

The refractive index of an irregularly shaped silica composite oxide particulate was measured using an Abbe refractometer (manufactured by ATAGO CO., LTD.) by a liquid immersion method (measurement wavelength: 589 nm). That is, in a thermostatic chamber at 25°C, 1 g of the composite oxide particle was dispersed in 50 mL of anhydrous toluene in a 100 mL sample bottle. 1-bromotoluene was gradually added dropwise while stirring this dispersion liquid with a stirrer, the refractive index of the dispersion liquid at the time point when the dispersion liquid became most transparent was measured, and the obtained value was used as a refractive index of the composite oxide particle.

### Examples 2 to 4 and Comparative Examples 1 to 5

Irregularly shaped silica-zirconia particulates F2 to F4 that correspond to the irregularly shaped silica composite oxide particulate according to the present invention and irregularly shaped silica-zirconia particulates F5 to F9 that do not correspond to the irregularly shaped silica composite oxide particulate according to the present invention were obtained in the same manner as that in Example 1 except that the raw materials and their usage amounts were changed as shown in Table 1. Note that the numerical value in the column of "Raw material" in Table 1 indicates the amount of each raw material used (unit: g (gram)). Further, in the column of "Proton concentration relative to reaction solution" and the column of "Water concentration relative to reaction solution" in Table 1, the value converted to the volume (L)-based concentration of a silica composite oxide precursor sol is shown in brackets [] for reference.

For F2 to F9 obtained in each of Examples and Comparative Examples, the B/A, the average particle diameter, the specific surface area, the pore volume, and the like were obtained in the same manner as that in Example 1. The results are shown in Table 2.

### Comparative Example 6

When the hydrolysis step was performed in the same manner as that in Example 1 except that the raw materials and their usage amounts were changed as shown in Table 1, a glassy solid precipitated during hydrolysis.

### Comparative Example 7

When the hydrolysis step to the gel preparation step were performed in the same manner as that in Example 1 except that the raw materials and their usage amounts were changed as shown in Table 1, the gelling of the sol did not occur.

### Comparative Example 8

Referring to the production method described in Patent Literature 1, an irregularly shaped silica composite oxide particulate F10 was synthesized using the following method. That is, 0.05 mass% hydrochloric acid: 5.4 g and TES: 160 g were dissolved in 0.2 L of 2-methyl-1-propanol (isobutyl alcohol) and mixed at room temperature for 5 hours to hydrolyze the mixture, and TBZ: 67 g and NaOMe: 7.7 g were added to the obtained alkyl silicate hydrolyzed solution and stirred for 1 hours, thereby obtaining a sol. Then, 30 g of water was added to the sol while stirring, thereby obtaining a wet gel. The obtained wet gel was allowed to stand at room temperature for 4 hours, the solidification thereof was confirmed, and then, collected as a composite oxide gel. Next, the composite oxide gel was air-dried at 100°C for 15 hours to dry and solidify. The dried and solidified gel was pulverized with a vibrating ball mill such that the average particle diameter would be 4 µm. After that, the pulverized product (powder) was fired at 900°C for 2 hours, thereby obtaining an irregularly shaped silica composite oxide particulate F10. The same analysis as that in Example 1 was performed on the F10 obtained in this way. The results are shown in Table 2.

### Example 5 and Comparative Examples 9 and 10

An irregularly shaped silica-titania particulate F11 that corresponds to the irregularly shaped silica composite oxide particulate according to the present invention and irregularly shaped silica-titania particulates F12 and F13 that do not correspond to the irregularly shaped silica composite oxide particulate according to the present invention were obtained in the same manner as that in Example 1 except that TPT was used as a metal raw material instead of TBZ and the raw materials and their usage amounts were changed as shown in Table 1. Note that the numerical value in the column of "Raw material" in Table 1 indicates the amount of each raw material used (unit: g (gram)). Further, for the obtained F11, the B/A, the average particle diameter, the specific surface area, the pore volume, and the like were obtained in the same manner as that in Example 1. The result are shown in Table 2.

**(Table 1)**

| | Name | Composite oxide raw material | | | | Condition and analysis result of each step | | | | | | | | |
|---|---|---|---|---|---|---|---|---|---|---|---|---|---|---|
| | | | | | | Hydrolysis step | | | | | | Sol preparation step | Gel forming step | Firing step |
| | | TES (g) | TBZ (g) | TPT (g) | NaOMe (g) | Proton concentration relative to reaction solution (mmol/L) | Water concentration relative to reaction solution (vol%) | Amount of protons relative to alkyl silicate (mol%) | Amount of water relative to alkyl silicate (mol%) | S₂/S₁ (%) | S3/S1 (%) | Precursor content ratio (mol/L) | Amount of mixed water (parts by mass)* | Firing temperature (°C) |
| Example 1 | F1 | 1056 | 190 | - | 51 | 0.63 [0.53] | 2.8 [2.4] | 0.026 | 64.6 | 37.3 | 4.6 | 2.3 | 7 | 900 |
| Example 2 | F2 | 1056 | 190 | - | 51 | 0.84 [0.71] | 3.7 [3.2] | 0.035 | 86.4 | 43.0 | 15.5 | 2.3 | 7 | 900 |
| Example 3 | F3 | 1056 | 222 | - | 51 | 0.63 [0.53] | 2.8 [2.4] | 0.026 | 64.6 | 37.3 | 4.6 | 2.3 | 7 | 900 |
| Example 4 | F4 | 1056 | 148 | - | 51 | 0.63 [0.54] | 2.8 [2.4] | 0.026 | 64.6 | 37.3 | 4.6 | 2.3 | 7 | 900 |
| Example 5 | F11 | 198 | - | 23 | 10 | 0.63 [0.53] | 2.8 [2.4] | 0,026 | 64.6 | 37.3 | 4.6 | 2.3 | 7 | 750 |
| Comparative Example 1 | F5 | 1056 | 190 | - | 51 | 0.43 [0.36] | 1.9 [1.6] | 0.018 | 43.8 | 27.3 | 1.4 | 2.3 | 7 | 900 |
| Comparative Example 2 | F6 | 1056 | 370 | - | 51 | 0.43 [0.34] | 1.9 [1.5] | 0.018 | 43.8 | 27.3 | 1.4 | 2.3 | 7 | 900 |
| Comparative Example 3 | F7 | 1056 | 190 | - | 102 | 0.43 [0.36] | 1.9 [1.6] | 0.018 | 43.8 | 27.3 | 1.4 | 2.4 | 7 | 900 |
| Comparative Example 4 | F8 | 1056 | 190 | - | 153 | 0.43 [0.35] | 1.9 [1.5] | 0.018 | 43.8 | 27.3 | 1.4 | 2.4 | 7 | 900 |
| Comparative Example 5 | F9 | 1056 | 190 | - | 25 | 0.43 [0.37] | 1.9 [1.6] | 0.018 | 43.8 | 27.3 | 1.4 | 2.3 | 7 | 900 |
| Comparative Example 6 | - | 1056 | 190 | - | 51 | 1.24 [1.05] | 5.5 [4.7] | 0.053 | 130.2 | - | - | 2.3 | - | - |
| Comparative Example 7 | - | 1056 | 190 | - | 51 | 0.63 [0.53] | 2.8 [2.4] | 0.026 | 64.6 | 37.3 | 4.6 | 2.3 | 4 | - |
| Comparative Example 8 | F10 | 160 | 67 | - | 8 | 0.20 [0.15] | 1.4 [1.1] | 0.010 | 39.0 | 40.5 | 2.0 | 2.0 | 7 | 900 |
| Comparative Example 9 | F12 | 198 | - | 23 | 10 | 0.63 [0.53] | 2.8 [2.4] | 0.026 | 64.6 | 37.3 | 4.6 | 2.3 | 7 | 800 |
| Comparative Example 10 | F13 | 198 | - | 23 | 10 | 0.43 [0.37] | 1.9 [1.6] | 0.018 | 43.8 | 27.3 | 1.4 | 2.3 | 7 | 750 |

| | | | | | | | | | | | | | | |
|---|---|---|---|---|---|---|---|---|---|---|---|---|---|---|
| *Refers to parts of mass of water with respect to 100 parts by mass of a silica composite oxide precursor sol | | | | | | | | | | | | | | |

**(Table 2)**

| | Name | Evaluation result of composite oxide particle | | | | | |
|---|---|---|---|---|---|---|---|
| | | Average particle diameter (µm) | Composition(mol%) SiO₂/ZrO₂/TiO₂/Na₂O | Specific surface area (m²g⁻¹) | Pore volume (cm³g⁻¹) | Refractive index | B/A |
| Example 1 | F1 | 4 | 90.00/7.66/0/2.34 | 4.154 | 0.048 | 1.518 | 0.78 |
| Example 2 | F2 | 4 | 90.00/7.66/0/2.34 | 4.230 | 0.045 | 1.504 | 0.63 |
| Example 3 | F3 | 4 | 88.88/8.82/0/2.31 | 4.332 | 0.042 | 1.520 | 0.71 |
| Example 4 | F4 | 4 | 91.57/6.06/0/2.38 | 4.599 | 0.043 | 1.512 | 0.51 |
| Example 5 | F11 | 4 | 90.00/0/7.66/2.34 | 3.368 | 0.038 | - | 0.58 |
| Comparative Example 1 | F5 | 4 | 90.00/7.66/0/2.34 | 4.529 | 0.047 | 1.532 | 1.03 |
| Comparative Example 2 | F6 | 4 | 84.06/14.35/0/1.59 | 4.300 | 0.045 | 1.548 | 2.38 |
| Comparative Example 3 | F7 | 4 | 87.94/7.49/0/4.57 | 4.182 | 0.037 | 1.528 | 4.00 |
| Comparative Example 4 | F8 | 4 | 85.98/7.32/0/6.70 | 3.361 | 0.035 | 1.530 | 4.00 |
| Comparative Example 5 | F9 | 4 | 91.06/7.76/0/1.18 | 30.547 | 0.151 | 1.500 | 0.64 |
| Comparative Example 8 | F10 | 4 | 81.71/16.16/0/2.12 | 4.500 | 0.045 | - | 1.68 |
| Cornparative Example 9 | F12 | 4 | 90.00/0/7.66/2.34 | 2.761 | 0.033 | - | 14.99 |
| Comparative Example 10 | F13 | 4 | 90.00/0/7.66/2.34 | 18.634 | 0.259 | - | 1.00 |

### 2. Preparation and evaluation of dental curable composition that includes irregularly shaped silica composite oxide particulate

The abbreviations of compounds used to prepare a curable composition are shown below.
<Polymerizable monomer>
   ·UDMA: 1,6-bis(methacrylethyloxycarbonylamino)trimethylhexane
   ·D-2.6E: 2,2-bis(4-methacryloyloxypolyethoxyphenyl)propane
   ·NPG: neopentyl glycol dimethacrylate
<Thermal polymerization initiator>
   ·BPO: benzoyl peroxide
<Spherical inorganic particulate having average particle diameter of 0.1 to 5 µm>
   ·PF1: γ-methacryloyloxypropyltrimethoxysilane-treated product of spherical silica-zirconia having average particle diameter of 0.5 µm
<Spherical inorganic particulate having average particle diameter of 0.01 to 0.1 µm>
   ·PF2: γ-methacryloyloxypropyltrimethoxysilane-treated product of spherical silica-titania having average particle diameter of 0.07 µm
<Irregularly shaped silica composite oxide particulate>
   ·FS1 to 13: γ-methacryloyloxypropyltrimethoxysilane-treated product of irregularly shaped silica composite oxide particulates F1 to 13 produced in Examples 1 to 5 and Comparative Examples 1 to 5, 8, 9, and 10.

### Example 6

UDMA: 15 parts by mass, D-2.6E: 70 parts by mass, NPG: 15 parts by mass, and BPO: 1.0 parts by mass were mixed to prepare a curable polymerizable monomer composition. Further, FS1: 60 parts by mass, PF1: 28 parts by mass, and PF2: 12 parts by mass were mixed to prepare an inorganic particulate composition. The above inorganic particulate composition and the above curable polymerizable monomer composition were mixed such that the inorganic particulate composition: 81 mass% and the curable polymerizable monomer composition: 19 mass% were obtained, thereby obtaining a uniform paste-like composition. After that, vacuum degassing was performed thereon to prepare a (dental) curable composition.

Next, a 14.5 mm × 14.5 mm × 18 mm mold was filled with the obtained curable composition, pressurized with nitrogen at 0.4 MPa in a pressure vessel, and heated for 15 hours while being allowed to stand in a heating device at 90°C to polymerize and cure. After cooling to room temperature, the cured body was removed from the mold. The obtained cured body was pressurized with nitrogen at 0.4 MPa in the pressure vessel again, allowed to stand in a heating device at 175°C, heat-treated for 48 hours, and then cooled to room temperature to obtain a dental resin material for cutting and machining. Evaluation of polishing properties and measurement of bending strength described below were performed. The results are shown in Table 3.

### (1) Evaluation of polishing properties

The outer surface of the obtained dental resin material for cutting was manually polished using waterproof abrasive paper P1500 to create a test piece. The test piece was placed on a dental workbench and sequentially polished in three stages using three types of polishing tools, and the gloss value of the polished surface after the completion of each polishing stage was measured. Note that the polishing in each stage was performed at a rotation speed of 10000 rpm for 30 seconds. Further, the measurement of a gloss value was performed using a gloss meter ("TC108D", manufactured by TokyoDenshoku. Co., Ltd.). The polishing tool used for each stage is shown below.
·Polishing tool used for the polishing in the first stage (brown silicon point polishing) : "Silicon Point M Type 2/HP/13", manufactured by SHOFU INC.
·Polishing tool used for the polishing in the second stage (blue silicon point polishing): "Silicon Point M Type 2/HP/13", manufactured by SHOFU INC.
·Polishing tool used for the polishing in the third stage (buff polishing): "an abrasive: SUPER STAR V", manufactured by NIPPON SHIKAKOGYOSHA Co., Ltd.

### (2) Measurement of bending strength

A 1.2 mm × 4.0 mm × 18 mm test piece was cut from the obtained dental resin material for cutting, and each surface of the test piece was finished with waterproof abrasive paper P2000. The prepared test piece was immersed in purified water and stored in a constant-temperature chamber at 37°C for 7 days, thereby obtaining a measurement test piece. For the measurement test piece, a three-point bending test was performed on a 4.0 mm × 18 mm surface using a universal testing machine Autograph (manufactured by Shimadzu Corporation) at room temperature in the atmosphere under the conditions of a distance between fulcrums of 12.0 mm and a crosshead speed of 1.0 mm/min. For each set of 10 test pieces, the bending strength [MPa] was calculated using the following formula and the average value was evaluated as bending strength. $Bending strength \left[MPa\right] = 3 FS / \left(2{bh}^{2}\right)$

Here, F: the maximum load [N] applied to the test piece, S: the distance [mm] between fulcrums, b: the width [mm] of the test piece measured immediately before the test, and h: the thickness [mm] of the test piece measured immediately before the test.

### Examples 7 to 10 and Comparative Examples 11 to 18

Curable compositions and dental resin materials for cutting were prepared in the same manner as that in Example 6 except that the composition of the inorganic particulate composition was changed as shown in Table 3. Further, for each of the obtained dental resin materials for cutting, evaluation of polishing properties and measurement of bending strength were performed in the same manner as that in Example 6. The results are shown in Table 3.

**(Table 3)**

| | Blending amount of inorganic particulate composition | | Polishing properties of cured body (gloss value (%)) | | | Bending strength (MPa) |
|---|---|---|---|---|---|---|
| | Spherical filler | Amorphous filler | Bronw silicone | Blue silicone | Buff polishing | |
| Example 6 | F1(28)/F2(12) | FS1(60) | 52.9 | 58.9 | 97.3 | 247 |
| Example 7 | F1(28)/F2(12) | FS2(60) | 58.2 | 59.3 | 99.6 | 240 |
| Example 8 | F1(28)/F2(12) | FS3(60) | 50.9 | 60.7 | 97.3 | 255 |
| Example 9 | F1(28)/F2(12) | FS4(60) | 50.2 | 60.4 | 95.1 | 250 |
| Example 10 | F1(28)/F2(12) | FS11(60) | 52.4 | 61.8 | 96.0 | 245 |
| Comparative Example 11 | F1(28)/F2(12) | FS5(60) | 46.2 | 53.3 | 90.3 | 250 |
| Comparative Example 12 | F1(28)/F2(12) | FS6(60) | 34.9 | 45.5 | 82.0 | 255 |
| Comparative Example 13 | F1(28)/F2(12) | F57(60) | 41.3 | 45.5 | 91.7 | 271 |
| Comparative Example 14 | F1(28)/F2(12) | F58(60) | 45.0 | 53.6 | 91.6 | 260 |
| Comparative Example 15 | F1(28)/F2(12) | FS9(60) | 49.8 | 57.6 | 96.7 | 223 |
| Comparative Example 16 | F1(28)/F2(12) | FS10(60) | 39.1 | 51.1 | 85.0 | 243 |
| Comparative Example 17 | F1(28)/F2(12) | FS12(60) | 43.7 | 45.8 | 73.3 | 250 |
| Comparative Example 18 | F1(28)/F2(12) | F513(60) | 54.9 | 65.6 | 97.9 | 210 |

As shown in Table 3, the dental compositions according to Examples 6 to 9, which includes the irregularly shaped silica-zirconia particulate according to the present invention, exhibited a high gloss value during polishing in each stage and a high level of bending strength. Further, as shown in Example 10, the irregularly shaped silica-titania particulate according to the present invention using TPT as a metal raw material also exhibited a high level of bending strength, and a cured body having a high gloss value during polishing was obtained.

On the other hand, in Comparative Examples 11 to 14, the gloss value was lower than those in Examples, although a high bending strength was exhibited. Similarly, in Comparative Example 17, a bending strength comparable to that in Example 10 was exhibited, but the gloss value during polishing was reduced. Further, in Comparative Example 15, an increase in gloss value was observed but the bending strength was reduced, because it is conceivable that the used irregularly shaped silica composite oxide particulate F9 has a large pore volume, i.e., 0.151 cm³/g and is formed of porous particles. Further, in Comparative Example 18, a high gloss value was obtained but the bending strength was reduced, because it is conceivable that it is formed of porous particles similarly to the above Comparative Example 15.

As shown in Comparative Example 16, in the case of using the irregularly shaped silica-zirconia particle described in Patent Literature 1, a high bending strength was exhibited, but the gloss value was lower than those in Examples.

### Reference Signs List

- 1: Halo peak derived from amorphous silica
- 2: Peak with highest diffraction intensity among diffraction peaks derived from zirconia

## Claims

1. An irregularly shaped silica composite oxide particulate that includes a non-porous silica composite oxide particle including silicon and at least one metal, **characterized in that**
a molar ratio of the at least one metal to a total amount of the silicon and the at least one metal in the silica composite oxide particle is 0.05 or more and 0.25 or less,
the irregularly shaped silica composite oxide particulate has an average particle diameter of 1 µm or more and 10 µm or less, which is defined as a median diameter in a volume-based particle size distribution obtained by a laser diffraction/scattering method,
in a diffraction pattern obtained by measuring X-ray diffraction for the irregularly shaped silica composite oxide particulate, a ratio B/A of diffraction intensity B at a peak top of a peak with the highest diffraction intensity among diffraction peaks derived from each oxide of the at least one metal to diffraction intensity A at a peak top of a halo peak derived from amorphous silica, which appears around 2θ = 22° is 0.5 or more and 0.9 or less.

2. The irregularly shaped silica composite oxide particulate according to claim 1, wherein
a specific surface area measured by a BET method is 3 m²g⁻¹ or more and 8 m²g⁻¹ or less, and in a pore distribution obtained by BJH method measurement, a total pore volume in a range of pore diameters of 10 nm or more and 50 nm or less is 0.06 cm³g⁻¹ or less.

3. The irregularly shaped silica composite oxide particulate according to claim 1, wherein
the at least one metal includes at least one of Zr or Ti and optionally Na.

4. A method of producing an irregularly shaped silica composite oxide particulate that includes a non-porous silica composite oxide particle including silicon and a metal that includes at least one of Zr or Ti, comprising:
a hydrolysis step of preparing a silica component raw material liquid that includes a hydrolysate of alkyl silicate and a condensed product of the hydrolysate by hydrolyzing at least part of the alkyl silicate in a reaction solution that includes an aqueous inorganic acid solution and a mixed solution of alkyl silicate and an alcohol,
a sol preparation step of preparing a precursor sol in which a silica composite oxide precursor particle that includes silicon and a metal that includes at least one of Zr or Ti is dispersed by mixing a metal raw material that includes at least one of alkyl zirconate and alkyl titanate with the silica component raw material liquid,
a gel forming step of forming a precursor wet gel by mixing 100 parts by mass of the precursor sol and 5 parts by mass or more and 10 parts by mass or less of water,
a pulverization step of obtaining an irregularly shaped precursor particulate by drying the precursor wet gel and then pulverizing it, and
a firing step of producing a non-porous silica composite oxide particle by firing the precursor particulate,
the method of producing an irregularly shaped silica composite oxide particulate being **characterized in that**
in the hydrolysis step,
(a) the alcohol is a branched alkyl alcohol having 3 to 5 carbon atoms,
(b) a concentration of protons contained in the aqueous inorganic acid solution relative to the reaction solution is 0.4 (mmol/L) or more and 0.9 (mmol/L) or less,
(c) a volume concentration of water in the reaction solution is 2 (vol%) or more and 4 (vol%) or less,
(d) a ratio of protons (mmol) contained in the aqueous inorganic acid solution to 1 (mmol) of the alkyl silicate is 0.02 (mol%) or more and 0.04 (mol%) or less,
(e) a ratio of an amount of water (mmol) contained in the reaction solution to 1 (mmol) of the alkyl silicate is 50 (mol%) or more and 100 (mol%) or less, and
hydrolysis and a condensation reaction are carried out using the reaction solution, and when, in a gas chromatogram obtained by performing gas chromatography analysis on the silica component raw material liquid prepared in the hydrolysis step, a peak area of a peak attributed to an unhydrolyzed product (a1) of alkyl silicate having no hydroxy group, which has at least some functional groups that may be substituted with alkoxy groups by the alcohol in the mixed solution, is defined as S₁, a peak area of a hydroxysilicate monomer (a2) that is a hydrolysate of the (a1), which has at least one hydroxy group and contains one silicon atom, is defined as S₂, and a peak area of a silicate dimer (a3) that is a condensed product of the (a2) and/or a condensed product of the (a1) and the (a2), which contains two silicon atoms, is defined as S₃, a peak area ratio of S₂ to S₁ is 0.3 or more and 0.6 or less and a peak area ratio S₃ to S₁ is 0.03 or more and 0.5 or less,
in the sol preparation step, the metal raw material and the silica component raw material liquid are mixed such that a molar ratio of the metal to a total amount of the silicon and the metal included in the silica composite oxide precursor particle is 0.05 or more and 0.25 or less and a content ratio of the silicon and the metal included in the silica composite oxide precursor particle to the precursor sol is 1.5 (mol/L) or more and 3 (mol/L) or less, and
in the firing step, the firing is performed within a range of 700°C or more and 1000°C or less.

5. The method of producing an irregularly shaped silica composite oxide particulate according to claim 4, wherein
the metal raw material includes a sodium alkoxide.

6. A dental curable composition, comprising:
a polymerizable monomer: 100 parts by mass;
a spherical inorganic particulate having an average particle diameter of 0.1 µm or more and 5 µm or less: 100 parts by mass or more and 200 parts by mass or less;
a spherical inorganic particulate having an average particle diameter of 0.01 µm or more and 0.1 µm or less: 40 parts by mass or more and 100 parts by mass or less;
the irregularly shaped silica composite oxide particulate according to claim 1: 100 parts by mass or more and 250 parts by mass or less; and
a polymerization initiator: 0.1 parts by mass or more and 1 part by mass or less.

7. A dental blank for cutting that includes a to-be-cut portion, **characterized in that**
at least part of the to-be-cut portion is formed of a cured body of the dental curable composition according to claim 6.
